Europäisches Patentamt

European Patent Office

Office européen des brevets

(11) Publication number: **0 457 738 A1**

# (12) EUROPEAN PATENT APPLICATION

(21) Application number: 91830207.6

(51) Int. Cl.⁵: **C07K 7/10, C12P 21/02**

(22) Date of filing: 17.05.91

(30) Priority: 18.05.90 IT 4797890

(43) Date of publication of application:
21.11.91 Bulletin 91/47

(84) Designated Contracting States:
AT BE CH DE DK ES FR GB GR LI LU NL SE

(71) Applicant: CONSIGLIO NAZIONALE DELLE
RICERCHE
Piazzale Aldo Moro, 7
I-00185 Roma (IT)

(72) Inventor: Porta, Raffaele, c/o CONSIGLIO
NAZIONALE
DELLE RICERCHE, Servizio Trasferimenti
Innovazioni
Via Tiburtina 770, I-00159 Rome (IT)

* Inventor: Ravagnan, Giampiero, c/o
CONSIGLIO NAZIONALE
DELLE RICERCHE, Servizio Trasferimenti
Innovazioni
Via Tiburtina 770, I-00159 Rome (IT)
Inventor: Peluso, Gianfranco, c/o CONSIGLIO
NAZIONALE
DELLE RICERCHE, Servizio Trasferimenti
Innovazioni
Via Tiburtina 770, I-00159 Rome (IT)
Inventor: Metafora, Salvatore, c/o CONSIGLIO
NAZIONALE
DELLE RICERCHE, Servizio Trasferimenti
Innovazioni
Via Tiburtina 770, I-00159 Rome (IT)

(74) Representative: de Simone, Domenico et al
Ing. Barzanò & Zanardo Roma S.p.A. Via
Piemonte 26
I-00187 Roma (IT)

(54) Di-aminated derivative of SV-IV protein and method for the preparation thereof.

(57) A derivative of the SV-IV protein, which has the two only glutamino residues of said protein in the 9 and 86 positions, each one of said residues being covalently bonded to a primary amine, and in particular to a polyamine which is preferably spermidine, and a process for the production of said derivatives; starting from the native SV-IV protein.

EP 0 457 738 A1

This invention relates to a di-aminated derivative of the SV-IV protein as well as to the process for the production thereof. More particularly, this invention consists in a derivative of the SV-IV protein wherein the gamma-carboxyamido groups of the two glutamino residues which are present in the native protein are so modified chemically as to yield a synthesis product that can be employed in the pharmaceutical field.

The SV-IV (SVS-IV) protein is a polypeptide which is synthesized and secreted in large amounts by the epithelium of the seminal vesicles of rat. Immunoreactive antigens correlated to said SV-IV protein have also been found in other organs of that species (lung, liver, uterus, brain) as well as in the human seminal liquid. The SV-IV protein is a basic protein having an isoelectric point of 8.9 and a low molecular weight ($Mr = 9753$), said protein being heat resistant, poorly soluble in water and polymorphic. Said protein has been purified to homogeneity from rat seminal vesicles by means of chromatographic separation procedures (Ostrowski, M.C., et al., 1979; The Journ. of Biol. Chem., 254, 383) and its amino acid sequence has been determined (Pan, Y.C.E., and Li, S.S.L., 1982, Int. J. Peptide Protein Res., 20, 177). The primary structure of the mature protein is made up of 90 amino acids, 2 of which being glutamino residues in the positions 9 and 86; the secondary structure is very low and, in vivo, the proteins seems not to be glycosylated.

The amount of the SV-IV protein which is present in the seminal vesicles increases after in vivo administration of testosterone; such increase depends on an increase in the number of mRNA molecules coding such protein in the epithelium cells, which fact thus puts into evidence an androgenous hormones-mediated regulation.

An investigation of the biological properties of the SV-IV protein has put into evidence that such protein shows immunosuppressive and anti-inflammatory properties (Metafora, S., et al., 1989, Biochemical Pharmacology, 38, 121).

The immunosuppressive activity is evidenced employing human or rat peripheral blood lymphocytes, normally in the quiescent state or splenocytes activated by mitogens. The presence of the SV-IV protein at low concentrations inhibits the proliferation of such immunocells as well as of a mixed lymphocyte culture. On the contrary, the anti-inflammatory activity has been put into evidence by observing a reduction in the carrageenin-induced oedema in rat following to administration of the SV-IV.

Moreover, an in vivo inhibition of the prostaglandin E2 biosynthesis has been observed, said inhibition being induced in rat peritoneal leucocytes by Bordetella pertussis, and an inhibition of the phospholipase $A_2$ activity was also observed in vitro. The biological properties mentioned above of the SV-IV protein make it a possible member of a whole family of proteins, i.e. the lipocortins, whose biosynthesis are controlled by steroid hormones, said proteins being characterized by an antiphospholipase $A_2$ activity.

Further investigations allowed to put into evidence that the SV-IV protein has an inhibiting activity as regards the synthesis of the platelet activating factor (PAF), (Camussi, G., Tetta, C. , Bussolino, F., Metafora, S., Peluso, G., Esposito, C. and Porta R., 1990, Eur. Journ. of Biochemistry, submitted for publication, and in addition it has a platelet antiaggregating activity (Persico, P., Mancuso, , F., Metafora, S., Peluso, G., Ravagnan, G., Esposito, C. and Porta, R., 1990, Biochem. Pharmacol., submitted for publication).

Finally it has been shown that said protein is a very good substrate of the transglutaminase enzyme (TGase) (Porta, R. et al., 1988, In "Advances in Post-translational Modifications of Proteins and Aging" Eds. Zappia, V., Galetti, P., Porta, R. and Wolf, F., p. 153-160, Plenum Press, New-York).

This last, in the presence of $Ca^{++}$ ions, catalyzes the formations of isopeptide covalent bonds between the $\gamma$-carboxamido groups of protein glutamino residues (glutamine: $HOOC\text{-}CHNH_2\text{-}CH_2\text{-}CH_2\text{-}CONH_2$), as acyl group donors, and various primary amines, in particular polyamines (putrescine, spermidine, spermine, monodansil-cadaverine, isoniazide, histamine, etc.) as acyl group acceptors, with the formation of protein $\gamma$-glutamylamines. Moreover, said TGase catalyzes the reaction between the $\gamma$-carboxamido groups of protein glutamino residues and $\varepsilon$-amino groups of protein lysine residue (lysine: $H_2N\text{-}CH_2\text{-}(CH_2)_3\text{-}CHNH_2\text{-}COOH$), with formation of $\varepsilon(\gamma\text{-glutamyl})$lysine crosslinks both within the polypeptide chain itself and between different polypeptide chains.

The biological properties of the SV-IV protein suggest a possible use of the same in the diagnostic and the pharmaceutical field for those pathological cases which are associated to abnormal immunoresponses and to inflammatory phenomena.

However, such applications are limited by the low solubility of the purified SV-IV protein, which is also a result of its self-aggregation ability. Moreover, as already mentioned above, the SV-IV protein, being a very good substrate of the transglutaminase, undergoes in vivo polymerization easily.

Accordingly, this invention proposes to provide an SV-IV protein derivative which is endowed with biological activities similar to those of the native protein and even better than those of the same, though free from the drawbacks mentioned above, so that this derivative can be employed advantageously in the pharmaceutical field.

To that aim, the property of the SV-IV protein has been exploited which consists in the capability for acting as a TGase acyl groups donor substrate for obtaining derivatives of the protein itself having two primary amine molecules covalently bonded to its two only glutamino residues in the 9 and 86 positions, so as to block the reactivity of the respective γ-carboxamido groups.

The derivatives of the type mentioned above show a higher solubility than the corresponding native protein as wella as an even higher immunosuppressive activity than the native protein itself.

Accordingly, it is a specific object of the present invention an SV-IV protein derivative which is characterized in that it has the two only glutamino residues of said protein in the 9 and 86 positions, each one being covalently bonded to a primary amine.

In particular, the primary structure of the derivative which is the object of the present invention is represented by the sequence SEQ ID 1.

Said primary amine is preferably a polyamine as for instance spermidine, spermine and putrescine.

According to a particular aspect of this invention, the di-aminated derivative of the SV-IV is obtained through the reaction with spermidine, which is a linear polyamine of the formula

$$NH_2-CH_2-CH_2-CH_2-NH-CH_2-CH_2-CH_2-CH_2-NH_2.$$

The derivatives according to the present invention are synthesized by means a process that is carried out according to the following steps: extraction of the SV-IV protein from rat seminal vesicles and purification of the protein itself; incubation of said protein in the presence of TGase, of a primary amine, of $CaCl_2$ and dithiothreitol; separation of the resulting derivative from the reaction mixture by means of cation exchange chromatography and filtering through an inert resin.

In such process, the extraction of the native protein from the rat seminal vesicle secretion and the purification of the protein itself are carried out according to the method described by Ostrowski et al., in the article mentioned above; the incubation step is carried out for a time ranging between 6 and 16 hours, at a pH between 7.5 and 9.0, and at a temperature between 25 and 40°C.

The derivative according to the present invention has shown an increase in water solubility which respect to the native protein; in addition, as such derivative has lost its capability of acting as a TGase acyl group donor substrate, it cannot be polymerized by the enzyme itself like the corresponding native SV-IV protein. Finally, such derivative shows an immunosuppressive activity remarkably higher than that of the SV-IV protein, while keeping the same anti-inflammatory property as well as the same platelet antiaggregating property and the property of inhibiting the synthesis of the platelet activating factor (PAF).

The properties mentioned above make such derivative employable in medical treatments for pathologies associated to abnormous immunoresponses such as leukaemias, lymphoms, auto-immune pathologies, allergies, anaphylactic shocks, organ transplantation rejection, and so on; for pathologies associated to inflammatory phenomena, like arthritis, rheumatoid arthritis, bacterial or viral infections, etc.; and for pathologies in connection with an abnormous platelet aggregation like athero-sclerosis, thrombosis, asthmatic bronchitis, and so on.

This invention will be disclosed now just for illustrative purposes in the following preparation example which is accompanied by the experimental results concerning the biological activity of the derivative so obtained.

Example

Synthesis and purification of the SV-IV

0.5 mg of the SV-IV purified to the homogeinity from the secretion of adult rat seminal vesicles (250-300 g) is incubated at 37°C for 12 hours in 1.0 ml of 125 mM Tris-HCl at pH 8.0 containing 2.5 mM $CaCl_2$, 10 mM dithiothreitol, and 0.2 M spermidine. A total of 50 µg of purified TGase from guinea pig liver (Sigma Chemical Co.) was added to the reaction mixture, said amount being divided into 5 aliquots of 10 µg each, at each 3-hr intervals during the 12-hr period of incubation. At the end of such incubation period, the reaction mixture was centrifuged at 10,000 rpm for 10 minutes and the supernatant was passed through a cation exchange chromatography column (Mono S HR 5/5) employing an automatic FPLC chromatograph (Pharmacia). The column was put in equilibrium with 50 mM sodium phosphate, pH 7.6 (solution A) and the column was eluted with the same solution containing 0.5 M NaCl (solution B). The derivative so obtained, which was designated as SV-IVp, was separated from the native SV-IV and from other modified forms of the protein by means of a linear gradient of the solution B from 0 % to 60 % in the first ten minutes (flowrate 1 ml/min), which was followed by a second linear gradient of the solution B from 60 % to 100 % in the next three minutes (flowrate 1 ml/min). The fractions containing the SV-IVp (the peak eluted after 13 minutes) were collected, pooled and passed through a small Sep-Pak $C_{18}$ column (Waters) put in equilibrium with 0.1 % trifluoroacetic acid (TFA) for removing the salts. The small column was washed with 5 ml of 0.1 % TFA, the SV-IVp was eluted with 3 ml of 70 % acetonitrile containing 0.1% TFA, and the eluate was taken to dryness in a vacuum centrifuge (Savant Speed Vac). The SV-IVp was redissolved by adding 50 mM Tris-HCl buffer at pH 7.5, at a concentration of 3 mg/ml. Such solution was stored at -20°C in aliquot parts which were defrosted in turn from time to time to perform the biological activity tests.

Biological Activities of the SV-IV protein

The immunosuppressive activity of the SV-IVp protein has been tested and compared to the activity of the native protein, by means of inhibition tests of the proliferation of human lymphocytes (PBL) after treatment with concanavalin A (Con A) in vitro.

The results of such experiments are shown in the following Table 1.

### Table 1

Effect of the native SV-IV protein and of the SV-IVp on the response to the Con A of human PBL

| Addition | | Incorporation of $(^3H)$ thymidine in lymphocytes (CPM ± S.E.M.) | |
|---|---|---|---|
| | | − Con A | + Con A |
| None | | 1,259 ± 240 | 51,815 ± 3,050 |
| native SV-IV | (5 µM) | 5,326 ± 450 | 29,431 ± 2,000 |
| " " " | (15 µM) | 1,250 ± 220 | 35,470 ± 2,350 |
| " " " | (25 µM) | 1,233 ± 210 | 46,675 ± 2,700 |
| SV-IVp (5 µM) | | 1,318 ± 250 | 28,735 ± 2,100 |
| " (15 µM) | | 1,526 ± 310 | 24,105 ± 1,900 |
| " (25 µM) | | 1,509 + 275 | 15,169 ± 1,000 |

EP 0 457 738 A1

It is evident from the table above that the SV-IVp protein shows a higher power of inhibiting the proliferation of human PBL which is induced in vitro by the Con A. Moreover, such derivative has lost its mitogenic activity in the absence of Con A, such activity being shown by the native protein at low concentrations (5 μM).

In a second series of experiments, 20 rats (body weight of about 300 g) were treated through the i.p. route 1 ml of PBS as modified by Dulbecco (Flow) 20 other rats were treated with 1 ml of PBS containing native SV-IV (1.5 mg/kg), and other 10 rats were treated with 1 ml of PBS containing the SV-IVp (1.5 mg/kg). Such administrations were repeated after 3 days. The animals were then sacrificed after another time of 3 days. The spleens of the animals that had been treated with SV-IVp showed a weight reduced to a half both with respect to the spleens from the control animals and to the spleens from the animals treated with native SV-IV. Moreover, the germinative centres of the spleens of the animals treated with SV-IVp showed to be less in number, when analyzed hystologically after dyeing with hematoxylin-eosine, both with respect to those present in the spleens of the control animals and to those present in the spleens of the animals treated with native SV-IV. From the sacrificed animals both the splenocytes (through trituration of spleens) and the PBLs (from the blood drawn by heart puncture) were prepared. Such cells were than tested on the basis of their response to the in vitro stimulations with Con A (according to the procedure described in Biochem. Pharmacol. 38, 121, 1989).

The results which are shown in the following Table 2, wherein $2 \times 10^5$ splenocytes and $3 \times 10^5$ PBLs/test were treated, put into evidence a lower response to the Con A by cells obtained from animals treated with SV-IVp.

## Table 2

Differential response to the in vitro stimulation with Con A of splenocytes and PBLs obtained from rats treated either with native SV-IV or with SV-IVp, with respect to control animals treated with a protein-free solution.

| Treatment | Incorporation of ($^3$H) thymidine in cells (cpm ± S.E.M.) | |
|---|---|---|
| | Splenocytes | PBL |
| Control rats | 15,000 ± 1,200 | 60,100 ± 2,550 |
| rats treated with native SV-IV | 9,500 ± 1,350 | 920 ± 105 |
| rats treated with SV-IVp | 4,500 ± 893 | 230 ± 75 |

The SV-IVp also has further biological properties which are fully similar to those of the native SV-IV, among which the following are mentioned in particular:

a) anti-inflammatory activity (cf. Biochem. Pharmacol. 38, 121, 1989);

b) platelet antiaggregating activity;

c) activity of inhibition of PAF synthesis.

This invention has been disclosed with specific reference to some preferred embodiments of the same, but it is to be understood that modifications and/or changes can be introduced by those who are skilled in the art without departing from the spirit and scope of the invention for which a priority right is claimed.

SPECIMEN SEQUENCE LISTING

| | |
|---|---|
| SEQ ID NO: | 1 |
| SEQUENCE TYPE: | amino acids |
| SEQUENCE LENGTH: | 90 amino acids (aa) |
| TOPOLOGY: | linear |
| MOLECULE TYPE: | modified protein |
| HYPOTHETICAL SEQUENCE: | no |
| FRAGMENT TYPE (for proteins only): | whole protein |
| ORIGINAL SOURCE: | seminal vesicles |
| ORGANISM: | rat |
| EXPERIMENTAL SOURCE: | SV-IV protein |

FEATURES:

amino acids 9 and 36, indicated as Xaa, represent glutamic residues covalently bonded with a primary amine at their gamma-carboxyamido group

PROPERTIES:

with respect to the unmodified protein SV-IV the protein is more soluble and has an increased immunosuppressive activity.

```
                     5                         10
      Arg-Lys-Thr-Lys-Glu-Lys-Tyr-Ser-Xaa-Ser-Glu-Glu-


             15                        20
      Val-Val-Ser-Glu-Ser-Phe-Ala-Ser-Gly-Pro-Ser-Ser-


      25                        30                        35
      Gly-Ser-Ser-Asp-Glu-Leu-Val-Arg-Asp-Lys-Pro-Tyr-


             40                        45
      Gly-Pro-Lys-Val-Ser-Gly-Gly-Ser-Phe-Gly-Glu-Glu-


             50                        55                        60
      Ala-Ser-Glu-Glu-Ile-Ser-Ser-Arg-Arg-Ser-Lys-His
```

                65                                    70
     Ile-Ser-Arg-Ser-Ser-Gly-Gly-Ser-Asn-Met-Glu-Gly-

                75                      . 80
     Glu-Ser-Ser-Tyr-Ala-Lys-Lys-Lys-Arg-Ser-Arg-Phe-

     85                      90
     Ala-Xaa-Asp-Val-Leu-Asn

## Claims

1. A derivative of the SV-IV protein, which is characterized in that the two only glutamino residues of said protein are present in its 9 and 86 positions, each one of said groups being covalently bonded to a primary amine.

2. A derivative according to claim 1, wherein said primary amine is a polyamine.

3. A derivative according to claim 2, wherein said polyamine is selected from the group made up of spermidine, spermine and putrescine.

4. A derivative according to claim 3, wherein said polyamine is spermidine.

5. A derivative of the SV-IV protein having the sequence SEQ ID 1.

6. A derivative according to claim 5 wherein said primary amine is a polyamine.

7. A derivative according to claim 5 wherein said polyamine is selected from the group made up of spermidine, spermine and putrescine.

8. A derivative according to claim 5 wherein said polyamine is spermidine.

9. A process for the production of derivatives according to claim 1-8, said process comprising the following steps: extraction of the SV-IV protein from rat seminal vesicles and purification of the same; incubation of said protein in the presence of TGase, of a primary amine, of $CaCl_2$ and dithiothreitol; separation of the resulting derivative from the reaction mixture by means of cation exchange chromatography and filtering through an inert resin.

10. A process according to claim 9 wherein said incubation is carried out for a time period in the range from 6 to 10 hours.

11. A process according to claim 9 or 10 wherein said incubation is carried out at a pH between 7.5 and 9.0.

12. A process according to claims 9, 10 or 11 wherein said incubation is carried out at a temperature between 25 and 40°C.

13. A process according to claim 9-12 wherein the TGase is divided into aliquot parts that are added to the reaction mixture at different times.

European Patent Office

# EUROPEAN SEARCH REPORT

Application Number

EP 91 83 0207

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | ADVANCES IN EXP. MED. AND BIOLOGY, vol. 250, 1988, pages 403-409; R. PORTA et al.: "Purification and structural characterization of in vitro synthesized (gamma-glutamyl) spermidine conjugates of a major protein secreted from the rat seminal vesicles" * The whole document, esp. page 4 * | 1-13 | C 07 K 7/10 C 12 P 21/02 |
| X | INT. J. PEPTIDE PROTEIN RES., vol. 35, February 1990, pages 117-122; R. PORTA et al.: "Transglutaminase-catalyzed modifications of SV-IV, a major protein secreted from the rat seminal vesicle epithelium" * Page 117 (abstract); page 118 * | 1-13 | |
| A | CHEMICAL ABSTRACTS, vol. 108, 1988, page 433, abstract no. 165168x, Columbus, Ohio, US; S. BENINATI et al.: "Covalent incorporation of polyamines as gamma-glutamyl derivatives into CHO cell protein", & BIOCHIM. BIOPHYS. ACTA 1988, 952(3), 325-33 * Abstract * | 7 | TECHNICAL FIELDS SEARCHED (Int. Cl.5) C 07 K C 12 P |

The present search report has been drawn up for all claims

| Place of search | Date of completion of the search | Examiner |
|---|---|---|
| THE HAGUE | 06-09-1991 | KORSNER S.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)